# EUROPÄISCHE PATENTANMELDUNG

(11) **EP 0 683 016 A1**
(43) Veröffentlichungstag der Anmeldung: **22.11.1995**
(21) Anmeldenummer: 95107415.2
(22) Anmeldetag: 16.05.1995
(51) Int. Cl.: B25J 3/00, B25J 18/06, A61B 19/00

(54) **Trägersystem aus in ihrer Raumform beliebig einstellbaren Rüsseln**

(30) Priorität: 18.05.1994 DE 4417400
(71) Anmelder: D.T.I. Dr. TRIPPE INGENIEURGES. mbH., D-76043 Karlsruhe (DE)
(72) Erfinder: Dorn, Jürgen, D-68809 Neulussheim (DE); Edinger, Wolfram, D-76199 Karlsruhe (DE); Trippe, Gustav, Dr., D-76133 Karlsruhe (DE)

(57) **Zusammenfassung**

Die Erfindung betrifft ein kraft- und bewegungssteuerbares Trägersystem für Aktoren, Sensoren, Sichtgeräte oder dergleichen und für den Transport von Medien.

Das System weist die nachstehenden Besonderheiten auf:
a) Das steuerbare Trägersystem umfaßt zwei baugleiche Rüssel (1,2),
b) jeder Rüssel ist aus einer Mehrzahl von in der Richtung seiner Längserstreckung aufeinanderfolgenden Segmenten gebildet,
c) aufeinanderfolgende Segmente sind gegeneinander abwinkelbar und in beliebig abgewinkelter Stellung arretierbar,
d) die die vorgenannten Abwinkelungen zulassende Anbindung aufeinanderfolgender Segmente aneinander ist im wesentlichen torsionssteif bezüglich Verdrehung um die Rüssel-Längsrichtung,
e) die Segmente sind einzeln oder in Gruppen durch an ihnen zugeordneten Krafteinleitungspunkten (5) angreifende äußere Verstellkräfte beaufschlagbar,
f) die Segmente sind innen hohl und folgen so aufeinander, daß der Rüssel die Grobkonfiguration eines Rohres mit einem durchgehenden inneren, sich in seiner Längsrichtung erstreckenden Hohlraum aufweist,
g) die Anbindung der Segmente aneinander erfolgt ausschließlich außerhalb des vorgenannten durchgehenden Hohlraums und im Bereich der gedachten Wandung der vorgenannten grobkonfigurierten Rohrform,
h) beide Rüssel bilden eine Anordnung aus Master und Slave, derart, daß die Kraft- und Bewegungssteuerung des einen (Slave) durch die gezielte Beeinflussung des anderen (Master) erfolgt, wobei die Bewegung des Master-Rüssels zeitgleich und identisch auf den Slave-Rüssel übertragbar ist und eine Rückkoppelung der auf den Slave-Rüssel einwirkenden Kräfte zum Master-Rüssel erfolgt.

## Beschreibung

Die Erfindung betrifft ein kraft- und bewegungssteuerbares Trägersystem für Aktoren, Sensoren, Sichtgeräte oder dergleichen und für den Transport von Medien.

Steuerbare, in ihrer Raumform veränderbare Gelenkarme kommen in vielen Technik-Bereichen zum Einsatz. Sie werden insbesondere im Zusammenhang mit Handhabungsautomaten oder Industrierobotern verwendet. In der Medizin-Technik verwendet man derartige Arme z. B. für endoskopische oder operative Zwecke.

Die Europäische Offenlegungsschrift 17 016 vermittelt einen Überblick über verschiedene, zum Stand der Technik gehörende Bauformen für Gelenkarme. Dabei sind zur Verstellung der insbesondere aufeinander abwälzenden Einzelelemente des jeweiligen Armes jedem dieser Einzelelemente mehrere hydraulisch, pneumatisch oder elektrisch betätigbare Servomotoren zugeordnet.
Die elektrisch betätigten Servomotoren ermöglichen lediglich eine Verstellung zwischen zwei Endpositionen. Daraus ergeben sich erhebliche Beschränkungen für die insgesamt von dem Arm einnehmbaren Raumformen.
Diese Beschränkungen werden z.T. überwunden durch die Verwendung hydraulisch betätigter Stellmotoren, deren Stellkolben stufenlos verschoben werden können. Dazu sind die verschiedenen Hydraulikzylinder gruppenweise an je eine Versorgungsleitung angeschlossen. Damit sind jedoch alle Hydraulikzylinder einer Gruppe stets nur mit einem allen gemeinsamen Druck beaufschlagbar, woraus sich neue Beschränkungen für die Verstellmöglichkeiten des Arms ergeben.

Bei einer anderen Ausführungsform nach der genannten EP-OS sind die Einzelelemente durch Zugdrähte miteinander verbunden und können gruppenweise durch Verkürzen oder Verlängern dieser Drähte in eine Winkelposition zueinander gebracht werden, woraus sich eine gekrümmte Raumform des gesamten Arms ergibt. Die Variationsmöglichkeiten für die Verstellung des gesamten Armes erscheinen auch hier beträchtlich eingeschränkt.

Aus der DE-PS 37 12067 ist ein beweglicher Arm bekannt, der aus einer Reihe von miteinander verbundenen gleichartigen Elementen besteht, die in Längsrichtung des Arms durch Gelenkachsen, insbesondere durch Kreuzgelenke allseitig miteinander verbunden sind, wobei zwischen jeweils zwei benachbarten Elementen ein zug- und druckfestes Raumgelenk sowie zwei Linearmotore angeordnet sind, die ebenfalls raumgelenkig an den benachbarten Gelenken angreifen. Die Linearmotore sind als doppeltwirkende Hydraulikzylinder ausgebildet. Als Steuereinrichtungen sind innerhalb der Elemente angeordnete Steuerventile vorgesehen. Ein solcher Arm weist ebenfalls erheblich beschränkte Verstellmöglichkeiten auf Zudem bedingt die Ansteuerung der in den Elementen untergebrachten Ventile einen beträchtlichen baulichen Aufwand.

Aus der DE-OS 3400362 ist ein Hydrogelenkarm bekannt, bei dem die hintereinander geschalteten Einzelelemente eine Anordnung aus mindestens drei periphären Zylindern und einem Zentralzylinder bilden, wobei sowohl eine Längenänderung als auch eine Beugung des Arms möglich ist. Die Zylinder befinden sich starr auf einer Grundplatte. Die peripheren Zylinder betätigen Kolbenstangen, die mit ihren Endgelenken in einem Schwenk-Schublagergehäuse verbunden sind. Bei der Beugung zweier Grundplatten zueinander findet eine Schiebebewegung in radialer Richtung, bezogen auf die Zentralzylinderachse, statt. Um die Verschiebung technisch zu ermöglichen wird eine Kurvenscheibe verwendet, die belegt ist durch den exzentrischen Zapfen eines Zahnrades, das durch eine Zahnstange bewegt wird, die mit einer Vorrichtung an die Kolbenstange des Zentralzylinders greift. Dieser Gelenkarm bietet zwar im Vergleich zu den zuvor beschriebenen erweiterte Möglichkeiten zur Verstellung des Arms im Raum - allerdings bedarf es eines ganz erheblichen baulichen Aufwands um diesen Vorteil zu erzielen. Die Vielzahl der benötigten Bauteile erlaubt einen besonders klein bauenden Arm, wie er zum Beispiel in der Medizin-Technik benötigt wird, nicht.

An ein zuverlässig arbeitendes, vielseitig verwendbares Trägersystem für Aktoren, Sensoren, Sichtgeräte oder dergleichen sind ganz besondere, aus dem jeweiligen besonderen praktischen Anwendungsfall erwachsende Anforderungen zu stellen, auf die nachstehend eingegangen wird.
Der Trägerarm sollte beliebige gekrümmte Raumformen einnehmen können, um sich mit seinem vorderen Ende an innerhalb seiner Reichweite liegende beliebige Raumpunkte unter einem beliebigen Winkel annähern zu können.
Er sollte zur Umfahrung von Hindernissen entlang nahezu beliebig dreidimensional gekrümmter Wege verfahrbar sein.
Die für einen derartigen Tragarm erforderliche Steuereinrichtung sollte in hohem Maße bedienungsfreundlich sein.
Eine Kraftrückkoppelung zum Bediener ist erwünscht, um diesem Informationen über an dem Trägerarm angreifende äußere Kräfte zu liefern.
Die Kraftrückkoppelung sollte sehr sensibel sein. Es ist daher zu fordern, daß Reibungseffekte entlang der verschiedenen Kraftübertragungswege bzw. aufgrund der verschiedenen Bewegungsabläufe minimiert sind.

Der Trägerarm sollte in seinen verschiedenen Betriebspositionen zuvorlässig formstabil gehalten werden können. Dazu ist es notwendig, ihn über seine ganze Länge bedarfsweise in einen biege- und insbesondere torsionssteifen Zustand versetzen zu können.

Besondere Anforderungen, auf die hier - da dem Fachmann geläufig - nicht näher eingegangen werden soll, ergeben sich aus speziellen Anwendungsfällen wie z.B. in menschenfeindlicher Umgebung (Radioaktivität, toxische Einflüsse, Explosionsgefahr, Untertageeinsatz, Unterwassereinsatz, Einsatz im Weltraum) oder bei beengten Raumverhältnissen, wenn menschlicher Zugang nicht möglich ist, insbesondere in der Medizintechnik, z.B. in der Minimal Invasiven Chirurgie (MIC) oder der Gehirnchirurgie.
In dem zuletzt genannten Anwendungsbereich ist der Zwang zur Miniaturisierung des Trägerarms außerordentlich groß, da Außendurchmesser von maximal 3 mm gefordert werden.

Der Erfindung liegt die Aufgabe zugrunde, ein Trägersystem anzugeben, das bei Überwindung der bei den verschiedenen, eingangs erwähnten bekannten Trägerarmen auftretenden Nachteile den vorstehend wiedergegebenen besonderen Anforderungen in hohem Maße gerecht wird, so daß es im Zusammenhang mit den unterschiedlichsten Anwendungsfällen möglichst universell eingesetzt werden kann. Besonderes Ziel der Erfindung ist dabei, das Trägersystem mit seinem Grundaufbau so vorzusehen, daß es in einem sehr hohen Maße miniaturisierbar ist, so daß es insbesondere auch für die in der Medizintechnik auftretenden komplexen technischen Aufgaben geeignet ist.

Die Lösung dieser Aufgabe erfolgt mit den im Patentanspruch 1 angegebenen Mitteln und Maßnahmen, also dadurch, daß das im Raum dreidimensional bewegbare, in seiner Raumform beliebig einstellbare kraft- und bewegungssteuerbare Trägersystem für Aktoren, Sensoren, Sichtgeräte oder dergleichen und für den Transport von Medien, die nachstehenden Merkmale aufweist:
a) Das steuerbare Trägersystem umfaßt zwei baugleiche Rüssel,
b) jeder Rüssel ist aus einer Mehrzahl von in der Richtung seiner Längserstreckung aufeinanderfolgenden Segmenten gebildet,
c) aufeinanderfolgende Segmente sind gegeneinander abwinkelbar und in beliebig abgewinkelter Stellung arretierbar,
d) die die vorgenannten Abwinkelungen zulassende Anbindung aufeinanderfolgender Segmente aneinander ist im wesentlichen torsionssteif bezüglich Verdrehung um die Rüssel-Längsrichtung,
e) die Segmente sind einzeln oder in Gruppen durch an ihnen zugeordneten Krafteinleitungspunkten angreifende äußere Verstellkräfte beaufschlagbar,
f) die Segmente sind innen hohl und folgen so aufeinander, daß der Rüssel die Grobkonfiguration eines Rohres mit einem durchgehenden inneren, sich in seiner Längsrichtung erstreckenden Hohlraum aufweist,
g) die Anbindung der Segmente aneinander erfolgt ausschließlich außerhalb des vorgenannten durchgehenden Hohlraums und im Bereich der gedachten Wandung der vorgenannten grobkonfigurierten Rohrform,
h) beide Rüssel bilden eine Anordnung aus Master und Slave, derart, daß die Kraft- und Bewegungssteuerung des einen (Slave) durch die gezielte Beeinflussung des anderen (Master) erfolgt, wobei die Bewegung des Master-Rüssels zeitgleich und identisch auf den Slave-Rüssel übertragbar ist und eine Rückkoppelung der auf den Slave-Rüssel einwirkenden Kräfte zum Master-Rüssel erfolgt.

Vorteilhafte Ausgestaltungen des erfindungsgemäßen Trägersystems sind in den Unteransprüchen wiedergegeben.

Die gefundene Lösung beruht insbesondere auf der Erkenntnis, daß die Verbindung einer Systemsteuerung nach Art von Master und Slave mit der im Anspruch 1 angegebenen besonderen konstruktiven Konzeption für die beiden baugleichen Rüssel in besonderem Maße dazu geeignet ist, der Erfüllung aller zuvor erwähnten Anforderungen in hohem Maße nahezukommen.
Der Master-Rüssel stellt die Bedienerschnittstelle dar, an der insbesondere aufgrund der Kraftrückkoppelung eine sensible Beeinflussung des Slave-Rüssels als Träger für Sensoren, Aktoren, oder Mittel für den Transport von Medien vorgenommen werden kann. Der Slave-Rüssel folgt dabei zeitgleich und in jeder Beziehung im Verhältnis 1 : 1 der Bewegung und Formveränderung des Master-Rüssels.

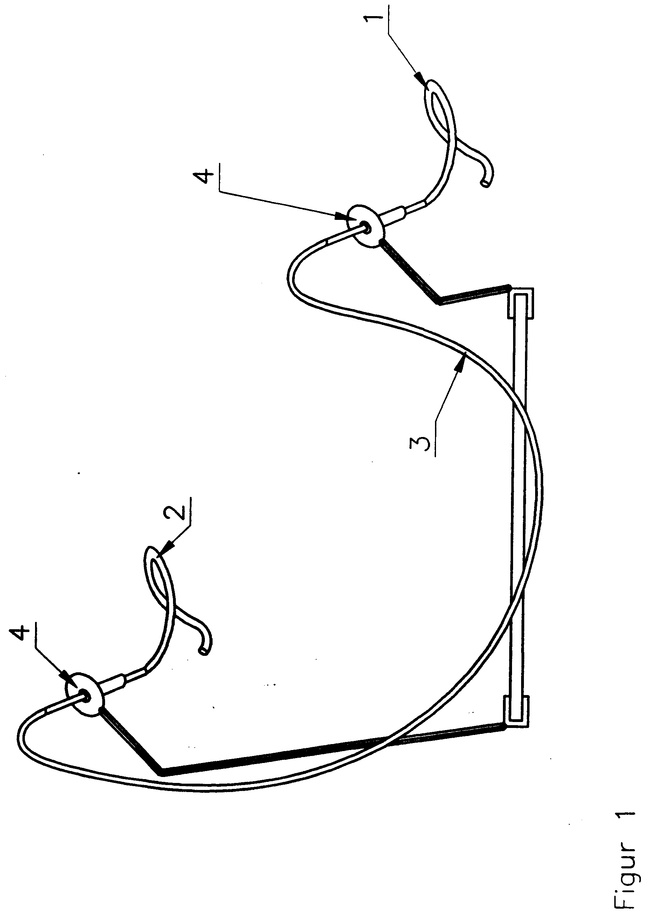

Im Inneren weisen beide Rüssel einen größtmöglichen freien Querschnitt auf, in dem insbesondere Signalleitungen, Werkzeugleistungsleitungen oder dgl. aufgenommen werden können. Außerdem bietet der freie Durchgang innen in jedem Rüssel die Möglichkeit, für den An- und Abtransport von Werkzeugen, Sensoren, Proben oder auch Medien.
Es kann festgestellt werden, daß gerade dieser Aufbau der Rüssel mit einem im Verhältnis zu ihrem Außendurchmesser außerordentlich großen freien Querschnitt im Inneren die Grundvoraussetzung für die Möglichkeit zu einer extrem hohen Miniaturisierung des erfindungsgemäßen Trägersystems bietet.
Steuerleitungen und -Organe können peripher und direkt an der Rüsselwandung anliegend angeordnet sein.
Das ausgenutzte Master-Slave-Steuerungsprinzip erlaubt ein synoptisches Steuern. Positioniert man Master und Slave, wie in Figur 1 der Zeichnung dargestellt, in gleicher Raumorientierung, ist die denkbar einfachste Bedienung möglich ohne irgendeine geistige Koordinatentransformation beim Bediener.

Die Formveränderung des Master-Rüssels erfolgt am einfachsten mit der Bedienerhand.

Anhand der Zeichnung ist das Trägersystem beispielhaft hinsichtlich einiger Besonderheiten näher zu erläutern.

Figur 1 zeigt schematisch beide Rüssel angeordnet an ihren Fixierungsstellen 4 und mit der Koppelungsleitung 4, über die eine Direktkoppelung oder auch eine indirekte Koppelung mit elektronischer Signalübertragung verwirklicht werden kann.

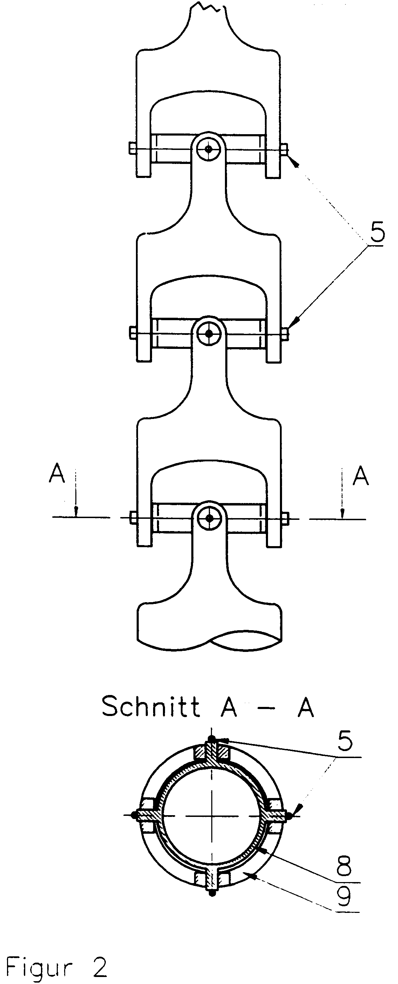

Figur 2 verdeutlicht die in den Ansprüchen 2 und 3 angegebene spezielle Bauform der Rüssel mit aufeinanderfolgenden Kreuzgelenkkörpern die über Gelenkringe 8 miteinander verbunden sind.

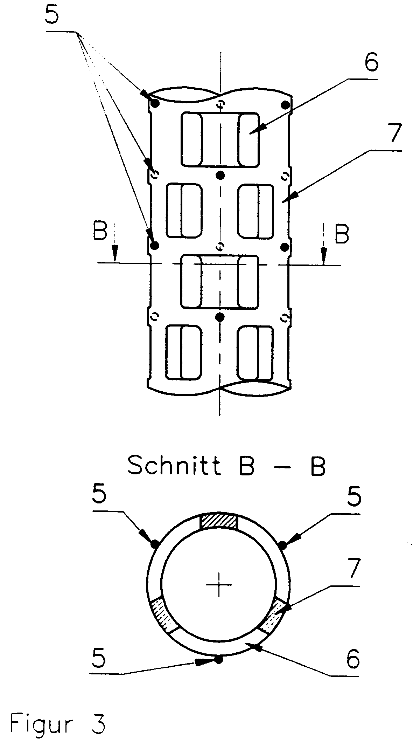

Aus Figur 3 ist der Aufbau eines Rüssels des Trägersystems als biegeelastisches Rohr mit Krafteinleitungspunkten 5, Ausnehmungen 6 und Stegen 7 ersichtlich. Das Rohr kann aus superelastischem Metall-Material, insbesondere aus einer Titan-Nickel-Legierung, gebildet sein. Unter superelastischem Material soll dabei solches mit einer außerordentlich hohen elastischen Dehnung von Epsilon bis zu 10 % verstanden werden.

| **Bezugszeichenliste** | |
|---|---|
| 1 | Master-Rüssel |
| 2 | Slave-Rüssel |
| 3 | Koppelungsleitung |
| 4 | Fixierungstelle |
| 5 | Krafteinleitungspunkt |
| 6 | Ausnehmung |
| 7 | Steg |
| 8 | Gelenkring |
| 9 | Jochteil |

## Patentansprüche

1. Kraft- und bewegungssteuerbares Trägersystem für Aktoren, Sensoren, Sichtgeräte oder dergleichen und für den Transport von Medien, mit im Raum dreidimensional bewegbaren, in ihrer Raumform beliebig einstellbaren Rüsseln, mit den nachstehenden Merkmalen:
a) Das steuerbare Trägersystem umfaßt zwei baugleiche Rüssel,
b) jeder Rüssel ist aus einer Mehrzahl von in der Richtung seiner Längserstreckung aufeinanderfolgenden Segmenten gebildet,
c) aufeinanderfolgende Segmente sind gegeneinander abwinkelbar und in beliebig abgewinkelter Stellung arretierbar,
d) die die vorgenannten Abwinkelungen zulassende Anbindung aufeinanderfolgender Segmente aneinander ist im wesentlichen torsionssteif bezüglichVerdrehung um die Rüssel-Längsrichtung,
e) die Segmente sind einzeln oder in Gruppen durch an ihnen zugeordneten Krafteinleitungspunkten (5) angreifende äußere Verstellkräfte beaufschlagbar,
f) die Segmente sind innen hohl und folgen so aufeinander, daß der Rüssel die Grobkonfiguration eines Rohres mit einem durchgehenden inneren, sich in seiner Längsrichtung erstreckenden Hohlraum aufweist,
g) die Anbindung der Segmente aneinander erfolgt ausschließlich außerhalb des vorgenannten durchgehenden Hohlraums und im Bereich der gedachten Wandung der vorgenannten grobkonfigurierten Rohrform,
h) beide Rüssel bilden eine Anordnung aus Master und Slave, derart, daß die Kraft- und Bewegungssteuerung des einen (Slave) durch die gezielte Beeinflussung des anderen (Master) erfolgt, wobei die Bewegung des Master-Rüssels (1) zeitgleich und identisch auf den Slave-Rüssel (2) übertragbar ist und eine Rückkoppelung der auf den Slave-Rüssel (2) einwirkenden Kräfte zum Master-Rüssel (1) erfolgt.

2. Trägersystem nach Anspruch 1, bei dem die aufeinanderfolgenden Segmente Kreuzgelenkkörper mit einem hohlen Mittelteil und daran in Rüssellängsrichtung beidendig angeschlossenen Jochteilen (9) sind. (Figur 2)

3. Trägersystem nach Anspruch 2, bei dem die Anbindung benachbarter Kreuzgelenkkörper untereinander über zwischen den Gelenkkörpern angeordnete Gelenkringe (8) mit Achsstummeln erfolgt, an denen die Jochteile (9) angelenkt sind.

4. Trägersystem nach Anspruch 1, bei dem die aufeinanderfolgenden Segmente ein durchgehendes biegeelastisches Rohr bilden, an dessen Wand in vorbestimmten, in Längsrichtung des Rüssels genommenen, den Segmentlängen entsprechenden Abständen Krafteinleitungspunkte (5) vorgesehen sind, über die die zwischen beiden Rüsseln wirkenden Kräfte übertragbar sind.

5. Trägersystem nach Anspruch 4, bei dem die Rohrwand in den Längenbereichen zwischen den Krafteinleitungspunkten (5) gleichmäßig über den Umfang verteilte Ausnehmungen (6) aufweist, zwischen denen in Längsrichtung des Rohres verlaufende Stege (7) gebildet sind. (Figur 3)

6. Trägersystem nach Anspruch 5, bei dem jeweils 3 über den Umfang verteilte Ausnehmungen (6) vorgesehen sind, zwischen denen 3 Stege (7) gebildet sind.

7. Trägersystem nach Anspruch 5 oder 6, bei dem in Längsrichtung aufeinanderfolgende Gruppen von Ausnehmungen (6) in Umfangsrichtung des Rohres mittig gegeneinander versetzt ausgebildet sind.

8. Trägersystem nach einem der Ansprüche 4 bis 7, bei dem das Rohr aus superelastischem Metall-Material, insbesondere einer Zusammensetzung mit Titan und Nickel, gebildet ist.

9. Trägersystem nach Anspruch 1, bei dem die Segmente als Rohrhülsen ausgebildet sind, deren Anbindung untereinander über zwischen den gegenüberliegenden Stirnflächen aufeinanderfolgender Rohrhülsen angeordnete elastische Ringe erfolgt.

10. Trägersystem nach Anspruch 1, bei dem die rohrförmige Grobkonfiguration mit einer Struktur nach Art einer Schraubenfeder gebildet ist, wobei jeweils eine vorbestimmte Anzahl von Windungen ein Segment bildet und die Anbindung benachbarter Segmente untereinander durch dazwischen liegende Windungen erfolgt.

11. Trägersystem nach einem oder mehreren der Ansprüche 1 bis 10, bei dem jeder der beiden Rüssel eine äußere Umhüllung aufweist, die aus einer Struktur nach Art einer Schraubenfeder gebildet ist.

12. Trägersystem nach einem oder mehreren der Ansprüche 1 bis 11, bei dem die beiden Rüssel direkt mechanisch gekoppelt sind.

13. Trägersystem nach Anspruch 12, bei dem die Verstellkräfte mittels Seilzügen auf die Segmente übertragen werden.

14. Trägersystem nach einem oder mehreren der Ansprüche 1 bis 11, bei dem die beiden Rüssel direkt hydraulisch gekoppelt sind.

15. Trägersystem nach Anspruch 14, bei dem die Verstellkräfte mittels Hydraulikzylindern auf die Segmente übertragen werden.

16. Trägersystem nach einem oder mehreren der Ansprüche 1 bis 11, bei dem die beiden Rüssel durch eine elektronische Signalübertragung gekoppelt sind und die Verstellkräfte mechanisch, insbesondere mittels Seilzügen, oder hydraulisch, insbesondere mittels Hydraulikzylindern, oder elektromagnetisch oder unter Nutzung anderer derartiger physikalischer Effekte auf die Segmente übertragen werden.
